(19) Europäisches Patentamt
European Patent Office
Office européen des brevets

(11) Numéro de publication : **0 336 817 B1**

(12) # FASCICULE DE BREVET EUROPEEN

(45) Date de publication du fascicule du brevet : **10.06.92 Bulletin 92/24**

(51) Int. Cl.$^5$ : **A61K 9/48,** C08B 37/04

(21) Numéro de dépôt : **89400867.1**

(22) Date de dépôt : **29.03.89**

(54) **Particules d'émulsion gélifiées et compositions les contenant.**

(30) Priorité : **29.03.88 FR 8804105**

(43) Date de publication de la demande : **11.10.89 Bulletin 89/41**

(45) Mention de la délivrance du brevet : **10.06.92 Bulletin 92/24**

(84) Etats contractants désignés : **AT BE CH DE ES FR GB GR IT LI LU NL SE**

(56) Documents cités :
**EP-A- 0 092 908**
**EP-A- 0 173 751**
**FR-A- 1 161 005**
**FR-A- 2 521 428**
**US-A- 3 563 769**
**US-A- 3 956 173**

(73) Titulaire : **HELENA RUBINSTEIN, INC.**
**202 Rodney Building 3411 Silverside Road**
**Wilmington Delaware 19810 (US)**

(72) Inventeur : **Clement, Anne**
**28, Avenue du Maine**
**F-75015 Paris (FR)**
Inventeur : **Fodor, Pierre**
**5, Rue du Professeur Victor Pauchet**
**F-92420 Vaucresson (FR)**
Inventeur : **Guth, Gérard**
**4, Avenue des Marronniers**
**F-95160 Montmorency (FR)**
Inventeur : **Thiollet Lilienthal, Nathalie**
**Institut Pasteur**
**B.P. 1274 Antananarivo (MG)**

(74) Mandataire : **Peuscet, Jacques et al**
**Cabinet Peuscet 68, rue d'Hauteville**
**F-75010 Paris (FR)**



Il est rappelé que : Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition (Art. 99(1) Convention sur le brevet européen).

Jouve, 18, rue Saint-Denis, 75001 PARIS

## Description

La présente invention a pour objet des compositions contenant des particules d'émulsion gélifiées.

Selon l'invention, la Demanderesse propose l'utilisation d'un nouveau type de présentation d'émulsions, dont la phase externe est aqueuse. Les particules d'émulsion selon l'invention sont gélifiées, rigidifiées au moins partiellement, individualisées en entités de formes diverses -et notamment en particules sphéroïdales-, et ce de manière réversible.

Cette particularité de leur présentation offre, quant à leur utilisation, de nombreux avantages.

Les particules d'émulsion gélifiées selon l'invention peuvent avantageusement être utilisées, selon les produits qu'elles contiennent pour des compositions utilisées, en cosmétologie, en pharmacie ou dans le domaine agro-alimentaire.

L'invention sera, dans la suite de la présente description, décrite plus particulièrement en référence au domaine cosmétique.

Pour des raisons de commodité d'emploi ou même pour de simples raisons esthétiques, on a déjà utilisé, pour des préparations cosmétiques, la présentation en capsules ou sphéres.

Lesdites préparations sont à l'intérieur d'une enveloppe ou capsule, qu'il est nécessaire de percer pour les recueillir et les appliquer.

Dans d'autres cas, on a proposé des préparations hétérogénes, pour inclure un ou plusieurs actifs dans un milieu chimiquement défavorable ou pour une libération de ces actifs au moment même de l'utilisation. Lesdits actifs sont emprisonnés dans un matériau adéquat qui est brisé par action mécanique.

S'il s'agit de microencapsulation, l'effet produit sur la peau est indétectable; dans le cas de capsules de plus grande taille, l'enveloppe demeure, avec les inconvénients que l'on peut imaginer.

Il existe, enfin, d'autres types de préparations cosmétiques, où des sphères ou gouttes de crème sont incluses dans un milieu gélifié, notamment transparent. Ceci confère à la préparation un aspect original sans pour autant que les sphères incluses puissent bouger sans risque de se mélanger au milieu gélifié.

La présente invention a pour objet des compositions pharmaceutiques, cosmétiques ou agroalimentaires, caractérisées par le fait qu'elles contiennent des particules d'émulsion gélifiées obtenables par addition et séjour au sein d'une solution contenant un agent gélifiant, de volumes prédéterminés, d'une émulsion à phase aqueuse externe, la phase aqueuse externe contenant en solution un réactif susceptible de former un gel, les particules d'émulsion gélifiées étant incluses dans un milieu contenant une base en quantité suffisante pour provoquer la fragilisation des particules d'émulsion gélifiées présentes. Dans ces compositions les particules gélifiés sont susceptibles de se mélanger très intimement à leurdit milieu d'inclusion, lors de la prise avec le doigt, puis étalement sur la peau ou par passage à travers un système approprié, tel un système de pompe.

Les particules d'émulsion gélifiées selon l'invention se caractérisent en ce qu'elles sont obtenables par addition et séjour au sein d'une solution gélifiante de volumes prédéterminés d'une émulsion à phase aqueuse externe, contenant en solution - dans ladite phase aqueuse un réactif susceptible de former un gel. Le séjour est plus ou moins prolongé selon le degré de gélification souhaité.

Les particules selon l'invention peuvent être obtenues à partir de toute émulsion dont la phase externe est aqueuse.

On entend par émulsion, dans la présente demande, tout type d'émulsions à phase externe aqueuse - macro- ou microémulsions, émulsions huile-dans-l'eau ou émulsions multiples, avec ou sans émulsionnant - ou tout autre système équivalent dont la phase externe est aqueuse, notamment les systèmes dispersés.

Ladite émulsion contient, en solution dans sa phase aqueuse, un réactif susceptible de former un gel. Il s'agit d'au moins un composé qui, au contact de la solution gélifiante, provoque la rigidification au moins partielle des volumes de l'émulsion introduits dans celle-ci.

Ladite solution gélifiante contient au moins un produit susceptible de réagir avec ledit réactif de l'émulsion.

La réaction mise en jeu est une réaction qui, à partir de deux composés solubles - respectivement dans l'émulsion à phase externe aqueuse et dans la solution gélifiante -, génère instantanément un insoluble.

Les particules d'émulsion sont piégées dans la structure de ce composé insoluble.

La consistance des entités insolubles obtenues est évidemment variable selon la nature et la concentration du réactif de l'émulsion et celles de la solution gélifiante.

Elle dépend également du temps de séjour desdites entités, desdits volumes dans ladite solution gélifiante.

En faisant varier ledit temps de séjour, on peut obtenir selon l'invention des produits différents :

– des produits, type gélules, gélifiés à leur périphérie seulement, sur une épaisseur plus ou moins importante ;

– des produits gélifiés, rigidifiés à coeur.

Ces différents types de particules font partie intégrante de l'invention. Les particules gélifiées à coeur en constituent toutefois une variante préférée.

De telles particules, gélifiées à coeur, sont avantageusement obtenues après maturation, c'est-à-dire qu'elles ont été laissées au contact de la solution gélifiante un temps suffisant pour que l'équilibre de la réaction mise en jeu soit atteint. Ledit équilibre étant atteint, les particules sont parfaitement stables.

L'addition de l'émulsion dans la solution gélifiante peut se faire à chaud ou à froid, selon différentes variantes. Elle se fait notamment à chaud avec des émulsions visqueuses telles que les crèmes. On peut faire varier la taille, la forme et la consistance des particules, en jouant sur de nombreux paramètres, et notamment sur la taille de l'orifice du système d'extrusion, sur la viscosité de l'émulsion, sa température d'extrusion, la concentration de la solution gélifiante...

Ainsi, en additionnant goutte à goutte l'émulsion, on obtient des particules sphéroïdales. Il s'agit là d'une variante préférée de l'invention.

On peut, selon d'autres variantes, obtenir des filaments ou autres entités de formes diverses.

Lesdites entités peuvent être recueillies par décantation ou filtration.

Elles peuvent également être conservées dans leur solution de préparation. Avantageusement, celle-ci contient alors des conservateurs.

Les composés solubles, respectivement dans la phase externe aqueuse de l'émulsion et dans la solution gélifiante et susceptibles de générer par contact un insoluble, sont de préférence des sels qui interagissent par échange d'ions.

Le réactif approprié dans l'émulsion à phase externe aqueuse est avantageusement choisi parmi des produits à structure polymérique et plus particulièrement parmi des produits à structure polysaccharidique portant des fonctions acides et leurs mélanges.

On utilise avantageusement, selon l'invention, des composés choisis parmi les alginates solubles (alginates de sodium par exemple); du carraghénane soluble , les dérivés de la chitine solubles ou leurs mélanges.

De tels composés interviennent généralement à raison de 0,2 à 1,5 % en poids dans l'émulsion. En fait, la quantité de réactif intervenant est fonction de la nature de celui-ci. Ainsi, par exemple, les alginates interviennent avantageusement à raison de 0,3 à 0,6 %, le carraghénane à raison d'environ 1 %...

Pour réagir avec lesdits produits, on utilise par exemple, dans la solution gélifiante, des sels solubles de métaux, notamment alcalins ou alcalino-terreux ou leurs mélanges. On utilise notamment du chlorure de calcium, de potassium...

Avantageusement, la solution gélifiante contient entre 0,01 et 0,05 mol/l de tels sels.

L'homme de l'art saura optimiser lesdites concentrations -concentration du réactif dans l'émulsion, concentration du produit appelé à réagir avec ledit réactif dans la solution gélifiante- pour l'obtention du résultat recherché.

Ainsi, des particules d'émulsion gélifiées peuvent avantageusement être obtenues par addition au sein d'une solution de chlorure de calcium de volumes prédéterminés, et notamment de gouttes, d'une émulsion à phase externe aqueuse, contenant de l'alginate de sodium. Lesdites particules sont récupérées, de préférence après maturation, par décantation ou filtration.

Le chlorure de calcium et l'alginate de sodium sont en solution. Ledit alginate de sodium au contact dudit chlorure de calcium génère un sel - alginate de calcium - dont la structure de type à liaisons croisées provoque la solidification de l'émulsion, plus précisément piège les particules de celle-ci.

Le délai au terme duquel les particules sont récupérées est avantageusement celui qui permet l'instauration d'un équilibre en ions calcium/sodium entre la solution gélifiante et lesdites particule: obtenues instantanément. Ce délai de maturation est d'environ 8 jours.

Les particules peuvent également être récupérées avant la fin de ce délai de maturation, leur degré de gélification étant alors moins avancé.

De la même façon, des particules d'émulsion gélifiées selon l'invention sont avantageusement obtenues par addition au sein d'une solution de chlorure de potassium de volumes prédéterminés et notamment de gouttes, d'une émulsion à phase aqueuse externe, contenant du carraghénane . De la même façon, les particules obtenues sont récupérées, de préférence après maturation, par décantation ou filtration.

La détermination des paramètres de cette manipulation - concentration en sel de la solution gélifiante, concentration en réactif de l'émulsion, présence d'additifs (éventuellement conservateurs), volume total de l'émulsion transférée dans la solution, volume de ladite solution utilisée, importance des volumes prédéterminés, temps de séjour... - est à la portée de l'homme du métier.

Il pourra aisément les faire varier pour obtenir des particules plus ou moins dures.

Comme précisé ci-dessus, on peut obtenir des particules gélifiées à partir de n'importe quelle émulsion à phase aqueuse externe. Notamment, on peut préparer des particules gélifiées avec des émulsions contenant des aminoacides, tels l'arginine, ou avec des émulsions contenant de la dihydroxyacétone, ou encore avec des émulsions contenant de la vitamine C.

En proposant une présentation originale à des émulsions à phase externe aqueuse, on améliore leur

stockage, leur introduction et leur conservation dans un autre milieu. Avantageusement, les particules gélifiées seront utilisées lorsque l'on veut stocker dans un même récipient(pour utilisation simultanée) des produits incompatibles entre eux.

Les particules gélifiées peuvent être obtenues avec des émulsions contenant différents types d'actifs. Il peut s'agir par exemple de principes actifs de médicaments, d'actifs de cosmétiques ou même de différentes matières premières du domaine agroalimentaire. Les particules gélifiées peuvent donc avantageusement être utilisées dans les industrie pharmaceutiques, agro-alimentaires ou cosmétiques.

On a indiqué précédemment que les particules d'émulsion gélifiées obtenables selon la présente invention peuvent être utilisées telles quelles ou introduites dans un milieu de viscosité variable. Leur relative rigidité autorise une telle utilisation.

Les compositions selon l'invention contiennent en fait, au moins une desdites particules et avantageusement de telles particules, obtenables à partir d'émulsions, de natures différentes.

Il est en effet, particulièrement intéressant d'utiliser des particules gélifiées, pour stocker dans un même milieu -dans une même composition- des volumes d'émulsions contenant des produits différents, notamment des produits incompatibles entre eux. Ces produits peuvent ainsi être stockés, conservés, ensemble, sans interaction avant une commune utilisation.

A titre d'exemple non limitatif, on cite la possibilité de préparer des particules d'émulsions contenant d'une part de la dihydroxyacétone et, d'autre part, des aminoacides tels que l'arginine ; d'inclure ces deux types de produits sous forme de particules gélifiées dans un milieu et de les y conserver sans qu'aucune réaction colorée ne soit observable.

Les compositions selon l'invention contenant les particules gélifiées peuvent consister en des solutions, gels ou émulsions.

Le terme "émulsion" a ici la même signification que précédemment : émulsion quelconque et notamment dispersion, à phase aqueuse externe.

Le gel peut être obtenu à partir de tout agent gélifiant, dispersible dans l'eau et plus particulièrement à partir d'agents gélifiants à structure carboxyvinylique (carbomers), à partir de polymères acryliques, de carboxyméthyl,-éthyl-, ou -propylcelluloses ou de gommes xanthanes.

Les particules gélifiées sont introduites dans lesdites compositions, par simple mélange. On peut en introduire, selon le le résultat recherché une plus ou moins grande quantité : par exemple de 5 à 50 % en poids, voire plus.

Les compositions, dans lesquelles sont incluses des particules d'émulsions gélifiées, contiennent selon l'invention une quantité suffisante de base, capable de produire une "réaction inverse" de celle qui a conduit à la gélification desdites particules (une réaction de "fragilisation" desdites particules).

On peut faire intervenir une base ou un mélange d'au moins deux bases.

La base est choisie parmi les bases compatibles avec le milieu, utilisables sans danger et susceptibles de générer à partir des particules gélifiées, insolubles, un nouveau composé soluble ; à partir notamment des sels insolubles, tel l'alginate de calcium ou le carraghénate de potassium, on génère un nouveau sel soluble. Elle sera de préférence choisie parmi les composés listés ci-après : la soude, la triéthanolamine, la diisopropanolamine, les aminoacides basiques tels que l'arginine, la lysine ; et leurs mélanges.

Pour obtention du résultat recherché, on fait généralement intervenir dans le milieu d'inclusion des particules, de 0,01 à 0,1 % en poids de base.

Ces chiffres sont toutefois donnés à titre indicatif ; l'homme de l'art saura, dans un cas particulier, optimiser ladite quantité de base.

On précise ici que, dans certains cas particuliers, le milieu d'inclusion desdites particules peut contenir une quantité de base beaucoup plus importante : ainsi, la quantité de base nécessaire pour fragiliser les particules peut s'ajouter à une certaine quantité de base nécessaire à la neutralisation du gel utilisé.

Les réactions mises en jeu pour la préparation et la fragilisation des particules de l'invention sont précisées ci-après, dans un cas particulier, pour l'exemple.

Au cours de la fabrication de particules, on peut mettre en jeu la réaction suivante :

$$\text{alginate de sodium} + CaCl_2 \longrightarrow \underline{\text{alginate de calcium}}\downarrow$$

dans lequel les particules de l'émulsion sont piégées.

Si les particules contenant ledit alginate de calcium sont incluses dans un milieu contenant de la triéthanolamine (TEA), une partie de celui-ci sera transformée en alginate de TEA, qui est un sel soluble. En conséquence, les particules à base d'alginate de calcium perdent en rigidité, se trouvent fragilisées.

On observe donc, en présence d'une base dans le milieu d'introduction des particules gélifiées selon l'invention, une diminution de leur rigidité, un retour au moins partiel de l'émulsion vers sa viscosité initiale.

En choisissant la nature de la base et en dosant sa teneur, on peut faire en sorte que les entités conservent leur forme et leur stabilité au sein du milieu, tout en devenant extrêmement souples. Elles sont alors susceptibles de se fondre dans ledit milieu pour générer un produit parfaitement homogéne sous une action mécanique quelconque. Ceci permet notamment, lors de la prise du mélange - milieu + particules gélifiées en son sein - et son étalement sur la peau, de reconstituer instantanément un produit parfaitement homogéne sans effort particulier.

Le mélange hétérogène - milieu + particules gélifiées en son sein - peut être utilisé dans un flacon pompe. Par simple action sur le mécanisme, on obtient un mélange extemporané, parfaitement homogéne.

De manière générale, un tel mélange peut avantageusement être utilisé dans un récipient muni d'un système de distribution tel qu'au passage au travers dudit système, ledit mélange hétérogéne devient homogéne : les particules d'émulsion gélifiées se fondant dans leur milieu d'inclusion.

D'autres avantages et caractéristiques de l'invention seront mieux compris à la lecture qui va suivre d'exemples de préparation de particules d'émulsion gélifiées selon l'invention et de leur incorporation dans un milieu adéquat.

I - Préparation des émulsions

Les pourcentages exprimés ci-après sont des pourcentages en poids.

EXEMPLE 1 : émulsion type lait.

On prépare deux phases A et B :

| | | | % |
|---|---|---|---|
| A | Acide stéarique | | 1,00 |
| | Alcool stéarylique | | 2,00 |
| | Isostéarate de glycéryle | | 3,50 |
| | Polysorbate 60 | | 1,20 |
| | Sesquioléate de sorbitanne | | 0,30 |
| | Huile minérale (et) alcool de lanoline | | 3,00 |
| | Diméthicone | | 1,00 |
| | Squalane | | 1,50 |
| B | Glycérine | | 5,00 |
| | Triéthanolamine | | 0,20 |
| | Alginate de sodium | | 0,50 |
| | Eau déminéralisée | q.s.p. | 100,00 |
| | Conservateur | | q.s. |
| C | Parfum | | q.s. |

On chauffe les phases A et B à 75°C. On prépare l'émulsion en versant sous agitation A dans B. On refroidit celle-ci lentement. Le parfum est ajouté à 40°C.

EXEMPLE 2 : émulsion type lait contenant de la dihydroxyacétone.

On prépare deux phases A et B :

| | | % |
|---|---|---|
| A | Tristéarate de sorbitanne | 0,50 |
| | Stéarate de PEG-40 | 1,25 |
| | Palmitate de cétyle | 3,50 |
| | Stéarate de glycéryle | 2,00 |
| | Triglycéride caprylique/caprique | 6,00 |
| | Diméthicone | 2,00 |
| | Tocophérol | 3,00 |
| | Ether stéarylique de PPG-15 | 5,00 |
| B | Glycérine | 5,00 |
| | Alginate de sodium | 0,40 |
| | Eau déminéralisée | q.s.p. 100,00 |
| | Conservateurs | q.s. |
| | Dihydroxyacétone | 10,00 |
| C | Parfum | q.s. |

Les phases A et B sont chauffées à 75°C. On prépare l'émulsion en versant sous agitation A dans B. On refroidit celle-ci lentement. Le parfum est ajouté à 40°C.

EXEMPLE 3 : émulsion type lait contenant de la L-arginine. On prépare deux phases A et B :

| | | % |
|---|---|---|
| A | Tristéarate de sorbitanne | 0,50 |
| | Stéarate de PEG-40 | 1,25 |
| | Palmitate de cétyle | 3,50 |
| | Stéarate de glycéryle | 2,00 |
| | Triglycéride caprylique/caprique | 6,00 |
| | Diméthicone | 2,00 |
| | Tocophérol | 3,00 |
| | Ether stéarylique de PPG-15 | 5,00 |
| B | Glycérine | 5,00 |
| | Alginate de sodium | 0,40 |
| | Eau déminéralisée | q.s.p. 100,00 |
| | Conservateurs | q.s. |
| | L-arginine | 10,00 |
| C | Parfum | q.s. |

Les phases A et B sont chauffées à 75°C.
On prépare l'émulsion en versant A dans B sous agitation.
On refroidit celle-ci lentement. Le parfum est ajouté à 40°C.

EXEMPLE 4 : émulsion type lait contenant un dérivé hydrosoluble de la vitamine C.

On prépare deux phases A et B :

| | | % |
|---|---|---|
| A | Tristéarate de sorbitanne | 0,50 |
| | Stéarate de PEG-40 | 1,25 |
| | Palmitate de cétyle | 3,50 |
| | Stéarate de glycéryle | 2,00 |
| | Triglycéride caprylique/caprique | 6,00 |
| | Diméthicone | 2,00 |
| | Ether stéarylique de PPG-15 | 5,00 |

| | | | |
|---|---|---|---|
| B | Glycérine | | 5,00 |
| | Alginate de sodium | | 0,40 |
| | Ascorbyl phosphate de magnésium | | 3,00 |
| | Eau déminéralisée | q.s.p. | 100,00 |
| | Conservateurs | | q.s. |
| C | Parfum | | q.s. |

Les phases A et B sont chauffées à 75°C.
On prépare l'émulsion en versant A dans B sous agitation.
On refroidit celle-ci lentement. Le parfum est ajouté à 40°C.

EXEMPLE 5 : émulsion type crème.

On prépare deux phases A et B :

| | | | % |
|---|---|---|---|
| A | Stéarate de PEG 30 et stéarate de glycéryle | | 6,00 |
| | Alcool stéarylique | | 3,00 |
| | Huile minérale | | 15,00 |
| B | Alginate de sodium | | 0,40 |
| | Glycérine | | 3,00 |
| | Triéthanolamine | | 0,35 |
| | Carbomer 934 | | 0,30 |
| | Eau déminéralisée | q.s.p. | 100,00 |
| | Conservateurs | | q.s. |
| C | Parfum | | q.s. |

Les phases A et B sont chauffées à 80°C.
On prépare l'émulsion en versant A dans B sous agitation.
On laisse refroidir à 50°C pour ajouter le parfum.
On conserve à 50°C. L'émulsion est utilisée à cette température, compte tenu de sa viscosité, pour être coulée dans la solution gélifiante (voir ci-après).

EXEMPLE 6 : Emulsion type crème.

On prépare deux phases A et B.

| | | % |
|---|---|---|
| A | Tristéarate de sorbitanne | 0,50 |
| | Stéarate PEG 40 | 1,25 |
| | Palmitate de cétyle | 3,00 |
| | Glycéryle monostéarate (auto émulsifiable) | 3,50 |
| | Huile minérale | 16,00 |
| B | Carraghénane de sodium | 1,00 |
| | Glycérine | 5,00 |
| | Eau déminéralisée | q.s. p 100,00 |
| | Conservateurs | q.s. |
| C | Parfum | q.s. |

Les phases A et B sont chauffées à 80°C

On réalise l'émulsion en les mélangeant sous agitation. Le parfum est ajouté à 50°C.

On conserve à 50°C. L'émulsion est utilisée à cette température, compte tenu de sa viscosité, pour être coulée dans la solution gélifiante (voir ci-après).

II - Préparation de la solution gélifiante

- Procédé utilisant le chlorure de calcium

| | |
|---|---|
| Chlorure de calcium | 3,33 g |
| Eau | q.s.p 1000 g |

- Procédé utilisant le chlorure de potassium

| | |
|---|---|
| Chlorure de potassium | 3,70 g |
| Eau | q.s.p 1000 g |

III - Préparation du gel dans lequel sont introduites les particules d'émulsion gélifiées selon l'invention

- Procédé utilisant un polymère carboxyvinylique

| | |
|---|---|
| Carbomer 940 | 0,60 g |
| Triéthanolamine | q.s.p pH 6,5 |
| Eau et Conservateurs | q.s.p 100 g |

- Procédé utilisant un polymère acrylique.

| | |
|---|---|
| Acrylate/steareth 20 | 3,00 g |
| PEG 20 | 5,00 g |
| Imidazolidinyl-urée | 0,30 g |
| Triéthanolamine | 0,30 g |
| Eau | q.s. p 100,00 g |

IV - Extrusion des particules

Les émulsions des exemples 1 à 6 ci-dessus sont introduites goutte à goutte dans la solution gélifiante.

Les émulsions des exemples 1 à 5 sont introduites dans la solution gélifiante contenant du chlorure de calcium ; l'émulsion de l'exemple 6 est introduite dans la solution gélifiante contenant du chlorure de potassium.

Pour cette introduction goutte à goutte, on utilise un récipient équipé d'un système à orifices multiples, pourvu que la vitesse d'introduction dans la solution gélifiante soit constante, condition d'obtention de particules de dimensions identiques.

L'opération est faite à froid (température ambiante) pour les émulsions de type lait. Si on utilise une crème, il faut travailler à chaud (50°C) pour ajuster la viscosité.

On décrit ci-après plus en détail cette opération, dans le cas de l'une quelconque des émulsions des exemples 1 à 5, pour illustration.

200 ml d'une émulsion sont coulés, goutte à goutte, dans 300 ml de la solution de chlorure de calcium à 0,03 mol/l.

A l'équilibre -au bout de 8 jours- on a une concentration équivalente en ions calcium dans les particules et dans la solution qui est égale à 0,018 mol/l. Dans ces conditions, les particules sont stables en rigidité et peuvent être conservées dans la solution de préparation, si celle-ci contient des conservateurs.

Les particules d'émulsion ainsi obtenues sont éventuellement introduites dans le gel.

On peut, à titre d'exemple, en introduire 20 % en poids.

Elles sont introduites dans le gel (polymère carboxyvinylique ou acrylique contenant de la triéthanolamine), après rinçage à l'eau déminéralisée.

L'homogénéité est obtenue grâce à une agitation lente ; on peut utiliser à cet effet, tout type de malaxeur disposant d'une ancre ou d'un système planétaire permettant d'ajuster la vitesse de rotation.

Des particules gélifiées selon l'invention, obtenues à partir des émulsions des exemples 2 et 3 ci-dessus, ont été incluses dans un même gel.

Aucune coloration n'est observée.

Les actifs ne réagissent pas l'un sur l'autre, même après plusieurs jours de vieillissement accéléré à l'étuve à 45°C.

Un produit témoin contenant aminoacide et dihydroxyacétone développe, quant à lui, une très rapide coloration.

On peut donc préparer avec les particules gélifiées selon l'invention un produit autobronzant très stable. Ledit produit est en fait préparé extemporanément par l'usager qui réalise par action mécanique le mélange des produits actifs.

## Revendications

### Revendications pour les Etats contractants suivants : AT, BE, CH, DE, FR, GB, IT, LI, LU, NL, SE

1. Compositions, notamment pharmaceutiques, cosmétiques ou agroalimentaires, caractérisées par le fait qu'elles contiennent des particules d'émulsion gélifiées susceptibles d'être obtenues par addition et séjour au sein d'une solution contenant un agent gélifiant, de volumes prédéterminés, d'une émulsion à phase aqueuse externe, la phase aqueuse externe contenant en solution un réactif susceptible de former un gel, les particules d'émulsion gélifiées étant incluses dans un milieu contenant une base en quantité suffisante pour provoquer la fragilisation des particules d'émulsion gélifiées présentes.

2. Compositions selon la revendication 1, caractérisée par le fait que la base est choisie parmi la soude, la triéthanolamine, la diisopropylamine et les aminoacides basiques.

3. Compositions selon l'une des revendications 1 ou 2, caractérisées par le fait que la quantité de base introduite dans le milieu d'inclusion des particules d'émulsion est de 0,01 à 0,1 % en poids.

4. Compositions selon l'une des revendications 1 à 3, caractérisées par le fait que les particules d'émulsion sont gélifiées au moins à leur périphérie.

5. Compositions selon l'une des revendications 1 à 4, caractérisées par le fait que les particules d'émulsion gélifiées sont gélifiées à coeur.

6. Compositions selon l'une des revendications 1 à 5, caractérisées par le fait que le réactif susceptible de former un gel est au moins un composé à structure polysaccharide portant des fonctions acides, de préférence un alginate, le carraghénane ou un dérivé de la chitine et leurs mélanges.

7. Compositions selon l'une des revendications 1 à 6, caractérisées par le fait que l'agent gélifiant est au moins un sel métallique, de préférence le chlorure de calcium ou de potassium.

8. Compositions selon l'une des revendications 1 à 7, caractérisées par le fait qu'elles contiennent des particules d'émulsion gélifiées obtenues à partir d'émulsions de natures différentes.

9. Compositions selon la revendication 8, caractérisées par le fait que l'une des émulsions contient de la dihydroxyacétone et une autre de la L-arginine.

10. Compositions selon l'une des revendications 1 à 9, caractérisées par le fait qu'elles sont conditionnées dans un récipient muni d'un système de distribution approprié, le passage de lesdites compositions au travers dudit système induisait la fusion des particules d'émulsion gélifiées dans leur milieu d'inclusion et la formation d'un mélange homogène.

### Revendications pour les Etats contractants suivants : ES, GR

1. Compositions, notamment cosmétiques ou agroalimentaires, caractérisées parle fait qu'elles contiennent des particules d'émulsion gélifiées susceptibles d'être obtenues par addition et séjour au sein d'une solution contenant un agent gélifiant, de volumes prédéterminés, d'une émulsion à phase aqueuse externe, la phase aqueuse externe contenant en solution un réactif susceptible de former un gel, les particules d'émulsion gélifiées étant incluses dans un milieu contenant une base en quantité suffisante pour provoquer la fragilisation des particules d'émulsion gélifiées présentes.

2. Compositions selon la revendication 1, caractérisée par le fait que la base est choisie parmi la soude, la triéthanolamine, la diisopropylamine et les aminoacides basiques.

3. Compositions selon l'une des revendications 1 ou 2, caractérisées par le fait que la quantité de base introduite dans le milieu d'inclusion des particules d'émulsion est de 0,01 à 0,1 % en poids.

4. Compositions selon l'une des revendications 1 à 3, caractérisées par le fait que les particules d'émulsion sont gélifiées au moins à leur périphérie.

5. Compositions selon l'une des revendications 1 à 4, caractérisées par le fait que les particules d'émulsion gélifiées sont gélifiées à coeur.

6. Compositions selon l'une des revendications 1 à 5, caractérisées par le fait que le réactif susceptible de former un gel est au moins un composé à structure polysaccharide portant des fonctions acides, de préférence un alginate, le carraghénane ou un dérivé de la chitine et leurs mélanges.

7. Compositions selon l'une des revendications 1 à 6, caractérisées par le fait que l'agent gélifiant est au moins un sel métallique, de préférence le chlorure de calcium ou de potassium.

8. Compositions selon l'une des revendicationa 1 à 7, caractérisées par le fait qu'elles contiennent des particules d'émulsion gélifiées obtenues à partir d'émulsions de natures différentes.

9. Compositions selon la revendication 8, caractérisées par le fait que l'une des émulsions contient de la dihydroxyacétone et une autre de la L-arginine.

10. Compositions selon l'une des revendications 1 à 9, caractérisées par le fait qu'elles sont conditionnées dans un récipient muni d'un système de distribution approprié, le passage de lesdites compositions au travers dudit système induisant la fusion des particules d'émulsion gélifiées dans leur milieu d'inclusion et la formation d'un mélange homogène.

11. Procédé pour la préparation de compositions pharmaceutiques, caractérisé par le fait qu'on prépare des particules d'émulsion gélifiées obtenables par addition et séjour au sein d'une solution contenant un agent gélifiant, de volumes prédéterminés, d'une émulsion à phase aqueuse externe contenant en solution un réactif susceptible de former un gel et que l'on inclut par mélange les particules d'émulsion obtenues dans un milieu contenant une base en quantité suffisante pour provoquer la fragilisation des particules d'émulsion gélifiées et un excipient pharmaceutiquement acceptable sous forme de solution, de gel ou d'émulsion à phase aqueuse externe.

## Patentansprüche

### Patentansprüche für folgende Vertragsstaaten : AT, BE, CH, DE, FR, GB, IT, LI, LU, NL, SE

1. Zusammensetzung, insbesondere für Pharmazie, Kosmetik und agrarische Nahrungsmittel. dadurch gekennzeichnet, daß sie Partikel einer Gelemulsion enthält, die dadurch erhältlich ist, daß man in eine Lösung, die ein gelbildendes Mittel enthält, vorgegebene Volumina einer Emulsion in äußerer wäßriger Phase hinzugibt und darin beläßt, wobei die äußere wäßrige Phase ein Reagenz in Lösung enthält, welches zur Ausbildung eines Gels befähigt ist und wobei die Partikel der Gelemulsion in ein Milieu eingeschlossen sind, welches eine Base in einer Menge enthält, die ausreicht, um die Versprödung der vorliegenden Partikel der Gelemulsion zu bewirken.

2. Zusammensetzung nach Anspruch 1, dadurch gekennzeichnet, daß die Base ausgewählt ist unter Soda, Triethanolamin, Diisopropylamin und basischen Aminosäuren.

3. Zusammensetzung nach einem der Ansprüche 1 oder 2, dadurch gekennzeichnet, daß die Menge der in das Einschlußmilieu der Emulsionspartikel eingeführten Base 0,01 bis 0,1 Gew.-% beträgt.

4. Zusammensetzung nach einem der Ansprüche 1 bis 3, dadurch gekennzeichnet, daß die Emulsionspartikel wenigstens an ihrer Peripherie geliert sind.

5. Zusammensetzung nach einem der Ansprüche 1 bis 4, dadurch gekennzeichnet, daß die Partikel der Gelemulsion im Inneren geliert sind.

6. Zusammensetzung nach einem der Ansprüche 1 bis 5, dadurch gekennzeichnet, daß das zur Gelbildung befähigte Reagenz mindestens eine Verbindung mit Polysaccharidstruktur ist, welche Säurefunktionen trägt, wie vorzugsweise ein Alginat, Carrageenan oder ein Chitinderivat oder ein Gemisch davon.

7. Zusammensetzung nach einem der Ansprüche 1 bis 6, dadurch gekennzeichnet, daß das gelbildende Mittel mindestens ein Metallsalz, vorzugsweise Calcium- oder Kaliumchlorid ist.

8. Zusammensetzung nach einem der Ansprüche 1 bis 7, dadurch gekennzeichnet, daß es Gelemulsionspartikel enthält, die erhältlich sind aus Emulsionen unterschiedlicher Natur.

9. Zusammensetzung nach Anspruch 8, dadurch gekennzeichnet, daß eine der Emulsionen Dihydroxyaceton und eine andere L-Arginin enthält.

10. Zusammensetzung nach einem der Ansprüche 1 bis 9, dadurch gekennzeichnet, daß sie in einem Behälter konditioniert ist, der mit einem geeigneten Abgabesystem versehen ist, wobei bei Durchgang der Zusammensetzung durch dieses System die Fusion der Partikel der Gelemulsion mit ihrem Einschlußmilieu und die Bildung eines homogenen Gemisches induziert werden.

### Patentansprüche für folgende Vertragsstaaten : ES, GR

1. Zusammensetzung, insbesondere für Kosmetik und agrarische Nahrungsmittel, dadurch gekennzeichnet, daß sie Partikel einer Gelemulsion enthält, die dadurch erhältlich ist, daß man in eine Lösung, die ein gelbildendes Mittel enthält, vorgegebene Volumina einer Emulsion in äußerer wäßriger Phase hinzugibt und darin beläßt, wobei die äußere wäßrige Phase ein Reagenz in Lösung enthält, welches zur Ausbildung eines Gels befähigt ist und wobei die Partikel der Gelemulsion in ein Milieu eingeschlossen sind, welches eine Base in einer Menge enthält, die ausreicht, um die Versprödung der vorliegenden Partikel der Gelemulsion zu bewirken.

2. Zusammensetzung nach Anspruch 1, dadurch gekennzeichnet, daß die Base ausgewählt ist unter Soda, Triethanolamin, Diisopropylamin und basischen Aminosäuren.

3. Zusammensetzung nach einem der Ansprüche 1 oder 2, dadurch gekennzeichnet, daß die Menge der

in das Einschlußmilieu der Emulsionspartikel eingeführten Base 0,01 bis 0,1 Gew.-% beträgt.

4. Zusammensetzung nach einem der Ansprüche 1 bis 3, dadurch gekennzeichnet, daß die Emulsionspartikel wenigstens an ihrer Peripherie geliert sind.

5. Zusammensetzung nach einem der Ansprüche 1 bis 4, dadurch gekenn-zeichnet, daß die Partikel der Gelemulsion im Inneren geliert sind.

6. Zusammensetzung nach einem der Ansprüche 1 bis 5, dadurch gekennzeichnet, daß das zur Gelbildung befähigte Reagenz mindestens eine Verbindung mit Polysaccharidstruktur ist, welche Säurefunktionen trägt, wie vorzugsweise ein Alginat, Carrageenan oder ein Chitinderivat, oder ein Gemisch davon.

7. Zusammensetzung nach einem der Ansprüche 1 bis 6, dadurch gekennzeichnet, daß das gelbildende Mittel mindestens ein Metallsalz, vorzugsweise Calcium- oder Kaliumchlorid ist.

8. Zusammensetzung nach einem der Ansprüche 1 bis 7, dadurch gekennzeichnet, daß es Gelemulsionspartikel enthält, die erhältlich sind aus Emulsionen unterschiedlicher Natur.

9. Zusammensetzung nach Anspruch 8, dadurch gekennzeichnet, daß eine der Emulsionen Dihydroxyaceton und eine andere L-Arginin enthält.

10. Zusammensetzung nach einem der Ansprüche 1 bis 9, dadurch gekennzeichnet, daß sie in einem Behälter konditioniert ist, der mit einem geeigneten Abgabesystem versehen ist, wobei bei Durchgang der Zusammensetzung durch dieses System die Fusion der Partikel der Gelemulsion mit ihrem Einschlußmilieu und die Bildung eines homogenen Gemisches induziert werden.

11. Verfahren zur Herstellung pharmazeutischer Zusammensetzungen, dadurch gekennzeichnet, daß man Partikel einer Gelemulsion herstellt, welche dadurch erhältlich sind, daß man in eine Lösung, die ein gelbildendes Mittel enthält, vorgegebene Volumina einer Emulsion in äußerer wäßriger Phase, die ein zur Gelbildung befähigtes Reagenz in Lösung enthält, hinzugibt und darin beläßt/und daß man die erhaltenen Emulsionspartikel durch Vermischen in ein Milieu einschließt, welches eine Base in einer Menge enthält, die ausreicht, um die Versprödung der Partikel der Gelemulsion zu bewirken, und einen pharmazeutisch verträglichen Exzipienten in Form einer Lösung, eines Gels oder einer Emulsion in äußerer wäßriger Phase enthält.

## Claims

### Claims for the following Contracting States : AT, BE, CH, DE, FR, GB, IT, LI, LU, NL, SE

1. Compositions, in particular pharmaceutical, cosmetic or food compositions, characterised in that they contain gelled emulsion particles capable of being obtained by adding to and maintaining in a solution containing a gelling agent, predetermined volumes of an emulsion with an aqueous external phase, the aqueous external phase containing in solution a reagent capable of forming a gel, the gelled emulsion particles being included in a medium containing a sufficient amount of base to render fragile the gelled emulsion particles present.

2. Compositions according to Claim 1, characterised in that the base is chosen from sodium hydroxide, triethanolamine, diisopropylamine and basic amino acids.

3. Compositions according to either of Claims 1 and 2, characterised in that the amount of base introduced into the emulsion particle inclusion medium is from 0.01 to 0.1 % by weight.

4. Compositions according to one of Claims 1 to 3, characterised in that the emulsion particles are gelled at least at their periphery.

5. Compositions according to one of Claims 1 to 4, characterised in that the gelled emulsion particles are gelled at the core.

6. Compositions according to one of Claims 1 to 5, characterised in that the reagent capable of forming a gel is at least one compound of polysaccharide structure, carrying acid functional groups, preferably an alginate, carrageenan or a chitin derivative and their mixtures.

7. Compositions according to one of Claims 1 to 6, characterised in that the gelling agent is at least one metal salt, preferably calcium or potassium chloride.

8. Compositions according to one of Claims 1 to 7, characterised in that they contain gelled emulsion particles obtained from emulsions of diverse nature.

9. Compositions according to Claim 8, characterised in that one of the emulsions contains dihydroxyacetone and another L-arginine.

10. Compositions according to one of Claims 1 to 9, characterised in that they are packaged in a container equipped with an appropriate dispensing system, the passage of the said compositions through the said system causing the fusion of the gelled emulsion particles in their inclusion medium and the formation of a homogeneous mixture.

**Claims for the following Contracting States : ES, GR**

1. Compositions, in particular cosmetic or food compositions, characterised in that they contain gelled emulsion particles capable of being obtained by adding to and maintaining in a solution containing a gelling agent, predetermined volumes of an emulsion with an aqueous external phase, the aqueous external phase containing in solution a reagent capable of forming a gel, the gelled emulsion particles being included in a medium containing a sufficient amount of base to render fragile the gelled emulsion particles present.

2. Compositions according to Claim 1, characterised in that the base is chosen from sodium hydroxide, triethanolamine, diisopropylamine and basic amino acids.

3. Compositions according to either of Claims 1 and 2, characterised in that the amount of base introduced into the emulsion particle inclusion medium is from 0.01 to 0.1 % by weight.

4. Compositions according to one of Claims 1 to 3, characterised in that the emulsion particles are gelled at least at their periphery.

5. Compositions according to one of Claims 1 to 4, characterised in that the gelled emulsion particles are gelled at the core.

6. Compositions according to one of Claims 1 to 5, characterised in that the reagent capable of forming a gel is at least one compound of polysaccharide structure, carrying acid functional groups, preferably an alginate, carrageenan or a chitin derivative and their mixtures.

7. Compositions according to one of Claims 1 to 6, characterised in that the gelling agent is at least one metal salt, preferably calcium or potassium chloride.

8. Compositions according to one of Claims 1 to 7, characterised in that they contain gelled emulsion particles obtained from emulsions of diverse nature.

9. Compositions according to Claim 8, characterised in that one of the emulsions contains dihydroxyacetone and another L-arginine.

10. Compositions according to one of Claims 1 to 9, characterised in that they are packaged in a container equipped with an appropriate dispensing system, the passage of the said compositions through the said system causing the fusion of the gelled emulsion particles in their inclusion medium and the formation of a homogeneous mixture.

11. Process for preparing pharmaceutical compositions characterised in that gelled emulsion particles are prepared which may be obtained by adding to and maintaining in a solution containing a gelling agent, predetermined volumes of an emulsion with an aqueous external phase containing in solution a reagent capable of forming a gel and in that the emulsion particles obtained are included, by mixing, in a medium containing a sufficient amount of base to render fragile the gelled emulsion particles and a pharmaceutically acceptable excipient in the form of a solution, a gel or an emulsion with an aqueous external phase.